# EUROPEAN PATENT SPECIFICATION

(11) **EP 1 638 472 B1**
(45) Date of publication and mention of the grant of the patent: **14.11.2012**
(21) Application number: 04744110.0
(22) Date of filing: 24.06.2004
(51) Int. Cl.: A61B 17/70

(54) **VERTEBRAL OSTEOSYNTHESIS EQUIPMENT**
WIRBEL-OSTEOSYNTHESE-GERÄT
DISPOSITIF D'OSTEOSYNTHESE VERTEBRALE

(30) Priority: 27.06.2003 FR 0307777; 29.07.2003 US 490520 P
(43) Date of publication of application: 29.03.2006
(73) Proprietor: Médicréa Technologies, 17000 La Rochelle (FR)
(72) Inventor: CLEMENT, Jean-Luc, F-06480 La Colle Sur Loup (FR); FIERE, Vincent, F-69006 Lyon (FR); TAYLOR, Jean, F-06400 Cannes (FR); ADAM, Yves, F-14280 Authie (FR); VILLARET, Bernard, F-17220 Croix-Chapeau (FR)
(74) Representative: Jeannet, Olivier
(86) International application number: PCT/IB2004/002458
(87) International publication number: WO 2005/000136

(56) References cited:
- DE-A- 19 512 709
- DE-C1- 3 701 765
- FR-A- 1 427 828
- FR-A- 2 743 290
- FR-A1- 2 342 714
- FR-A5- 2 226 877
- US-A- 4 607 547
- US-A1- 2003 073 996
- US-B1- 6 267 765

## Description

This patent application claims the priorities to :
- FR 03 07777, filed on June 27, 2003 ;
- US Provisional application N° 60/490,520, filed on July 29, 2003.

The present invention relates to vertebral osteosynthesis equipment and a manufacturing process of a bony anchoring member included in such equipment.

A vertebral osteosynthesis equipment generally includes bony anchoring members, such as pedicular screws or lamar hooks, one or two linking rods, intended to be connected to these anchoring members and to be attached to the vertebrae by dint thereof, and parts for connecting this(these) linking rod(s) to these anchoring members. The equipment may also comprise length-adjustable crossbeams, which link transversally two parallel linking rods in order to hold said rods with respect to one another.

In an existing type of equipment, such as the one disclosed in the document US 6 267 765, each anchoring member comprises a proximal threaded stud whereon a nut may be screwed, and each connecting part comprises a rounded section intended for surrounding a linking rod and two parallel drilled wings. These wings are intended for engaging onto said proximal threaded stud and for being clamped, by means of that nut, against a bearing surface provided on the anchoring member, said operation enabling to clam said rounded section around the linking rod and thereby ensuring longitudinal immobilisation of this rod with respect to the anchoring member. The anchoring members may be of "monoaxial" type, i.e. comprise a proximal threaded stud integral with of the portion of the anchoring member intended for gripping the bone, or may be of "polyaxial" type, i.e. comprise a proximal threaded stud articulated with respect to that portion intended for gripping the bone.

A polyaxial anchoring member known comprises, for the purpose of articulation of said proximal stud, a spherical head integral with this stud, received in a corresponding spherical cavity. In several types of existing anchoring members, this cavity is formed by providing a spherical semi-cavity in the proximal zone of the anchoring member and a portion of spherical cavity in the internal zone of a shouldered nut which is screwed on this proximal zone, this nut and this proximal zone locking said spherical head therebetween.

The shortcoming of the anchoring members known is that, after implantation, they protrude high above the bony zone whereon they are placed. These significant heights, if they are admissible for treating certain locations of a vertebral spine, in particular the lumbar vertebrae or the lumbar sacral articulation, may prove disturbing, let alone unacceptable, for treating other segments of a vertebral spine, in particular dorsal vertebrae, or for implanting particular equipment such as equipment placed laterally on the vertebrae to be treated.

A polyaxial anchoring member known comprises moreover a proximal gripping portion enabling of hold said member when clamping the nut. This proximal gripping portion, notably in the form of a hexagonal zone intended for co-operating with a corresponding holding key on known equipment, contributes to confer significant height to the anchoring member.

The purpose of the present invention is to remedy the shortcomings resulting from the height of a polyaxial anchoring member above the bony zone whereon this anchoring member is placed.

The vertebral osteosynthesis equipment affected comprises bony anchoring members, such as pedicular screws and/or lamar hooks, one or two linking rods, intended to be connected to these anchoring members and to be attached to the vertebrae by dint thereof, parts for connecting this(these) linking rod(s) to these anchoring members, and clamping means, such as nuts, for locking the linking rod(s) in said connecting parts ; at least one of said bony anchoring members is of the "polyaxial" type, i.e. comprises one proximal stud articulated with respect to a body intended for gripping a vertebra. The pre-characterizing part of claim 1 is known from US 6 267 765. According to the invention,
- the proximal stud of said polyaxial anchoring member exhibits an articulation head in the form of a spherical cap ; and
- said body of this anchoring member comprises a cavity intended for accommodating this articulation head and a wall surrounding this cavity, this wall being crimped around said articulation head and shaped in order to provide a proximal form, at least partially hemispherical.

The anchoring member according to the invention does not comprise therefore any spherical head no shouldered nut for capturing this spherical head but an articulation head in the form of a spherical cap retained in an articulation cavity by a wall crimped around said cavity.

The height of the portion of the anchoring member according to the invention which protrudes from a vertebra after implantation is, consequently, quite notably reduced with respect to the height of the anchoring members according to prior techniques.

The method according to the invention comprises the steps consisting in :
- providing, on the part intended for the proximal stud of said polyaxial anchoring member, an articulation head in the form of a spherical cap ;
- providing a cavity in the proximal zone of the part intended for said body of said anchoring member polyaxial, and, around this cavity, a wall which may be crimped ;
- engaging said articulation head into said cavity, and
- crimping said wall around said articulation head so that this wall exhibits a proximal form at least partially hemispherical.

Said body comprises advantageously a proximal gripping portion enabling to hold said body when tightening said clamping means, this proximal gripping portion being formed of a collar exhibiting several radial notches, notably four notches at 90° to one another.

This proximal gripping portion exhibits a height which is noticeably smaller than that of a hexagonal zone according to the prior technique, and thus contributes to limiting the height of the anchoring member.

Preferably, the equipment according to the invention comprises at least one connecting part exhibiting a rounded section intended for surrounding a linking rod and two parallel drilled wings, these wings being intended for engaging onto said proximal stud and for being clamped, using said clamping means, against a bearing surface contained in said polyaxial anchoring member; the distal wing of this connecting part exhibits moreover a distal cavity in the form of a spherical cap, of greater diameter than that of said crimped wall, the connecting part being intended for resting against this crimped wall at of this cavity.

This cavity, its diameter being greater than that of said crimped wall, enables to lock said proximal stud in an angular position with respect to the body of the anchoring member.

Advantageously, said clamping means is a nut and the proximal branch of this connecting part comprises a proximal cavity wherein a corresponding zone exhibited by this nut is intended for engaging.

After assembly, the nut is thus partially effaced in this cavity, which contributes further to reducing the height of the equipment above a vertebra, after implantation.

Preferably, this cavity, and said corresponding zone of the nut, are conical in shape, in order to increase the bearing surface of the nut against the connecting part.

The proximal stud and said connecting part may comprise means enabling to immobilise the proximal stud in rotation when the connecting part is engaged on this proximal stud. These means may in particular be at least one flat surface provided on the proximal stud and at least one flat surface provided on the connecting part, whereas these flats surfaces are immediately close to one another when the connecting part is engaged on the proximal stud.

The invention will be better understood, and other characteristics and advantages thereof will appear, with reference to the appended schematic drawing, representing, for non-limiting exemplification purposes, a preferred embodiment of parts included in the equipment affected.
Figure 1 is a partial view, before assembly, of a polyaxial pedicular screw, of a linking rod, represented as seen from its end, and, as a cross sectional view, of a connecting part and of a nut enabling to assemble a linking rod to this screw ; and
Figure 2 is a view of these parts similar to Figure 1, after assembly.

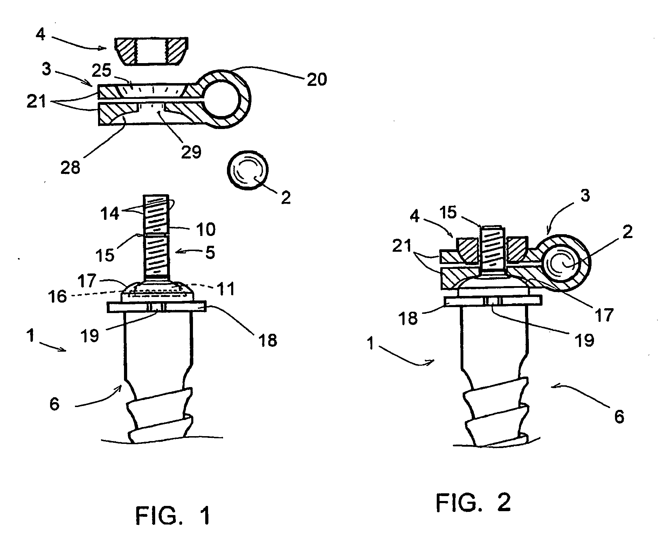

Figure 1 represents a polyaxial pedicular screw 1, a linking rod 2 for connecting several of these screws 1, a part 3 for connecting this rod 2 to one of these screws 1 and a nut 4 enabling to assemble the linking rod 2 to this screw 1.

The screw 1 comprises a proximal threaded stud 5 and a threaded distal screw body 6. The stud 5 is intended for receiving the part 3 engaged thereon and the nut 4 screwed thereon while the body 6 is intended for insertion into the pedicula of a vertebra.

The stud 5 exhibits a threaded cylindrical portion 10 and an enlarged distal head 11.

The portion 10 exhibits a zone 15 of reduced diameter, enabling to break its proximal portion after placing and clamping the nut 4, as appears by comparison of Figures 1 and 2.

This portion 10 also comprises two lateral flat surfaces 14.

The head 11 exhibits a diameter of the order of double the diameter of the portion 10 and looks like a spherical cap. This head 11 is intended for engaging in a proximal cavity 16 delineated by the proximal zone of the body 6 and for retention in this cavity 16 by crimping a proximal wall 17 exhibited by this body 6 around the cavity 16. After crimping, the wall 17 is shaped in order to have a proximal hemispherical form. As shown on Figure 1, the dimensions of the cavity 16 and of the aperture delineated by the wall 17 after crimping to let through the stud 5 are such that a multidirectional backlash of this stud 5 with respect to the body 6 is possible.

The body 6 also comprises a proximal collar 18, intended for abutting against the pedicula of the vertebra treated. This collar 18 exhibits several radial notches 19, notably four notches at 90° to one another, for holding the body 6 in rotation when clamping the nut 4.

The linking rod 2 is cylindrical and exhibits such rigidity as to hold several vertebrae with respect to one another. This rod 2 is however deformable in order to be shaped relative to the correction of the rachis to be performed.

The connecting part 3 comprises a rounded section 20 intended for surrounding the linking rod 2 and two parallel lateral wings 21 drilled with holes for engaging the part 3 on the stud 5. These wings 21 are distant mutually so that, in a distant position, the rod 2 may be inserted and may slide in the portion 20, and that, in a close position provided by the clamping of the nut 4, they clamp the portion 20 around the rod 2, immobilising the latter with respect to the part 3.

As shown on Figures, the proximal wing 21 exhibits a proximal pan 25 whereof the shape is suitable for the nut 4 to rest on, the latter exhibiting a corresponding conical zone. The distal wing 21 exhibits, for its own part, a distal cavity 28 in the form of a spherical cap, whereof the diameter is greater than that of the wall 17, and a hole 29. This hole 29 comprises two flat surfaces whereof the distance is slightly greater than that between both flat surfaces 14, so that when the stud 5 is engaged in this hole, the stud 5 is immobilised in rotation with respect to the part 3.

In practice, the number of screws 1 necessary to the treatment to be performed is placed in the pediculae of the vertebrae affected, then the connecting parts 3, with the rod 2 engaged in the portions 20, are placed on the studs 5. The nuts 4 are then clamped to immobilise the rod 2 with respect to the parts 3 and the proximal portions of the studs 5 are cut off.

As shown by the foregoing, the invention provides a vertebral osteosynthesis equipment whereof the anchoring members only protrude marginally beyond the vertebrae wherein they are implanted and are, consequently, only little sensitive under the skin, or even not sensitive at all. Such equipment may thus be used for treating not only lumbar vertebrae or the lumbar sacral articulation, but also dorsal vertebrae or for implanting equipment placed laterally on the vertebrae to be treated.

It is obvious that the invention is not limited to the embodiment described above for exemplification purposes but that it extends to all the embodiments covered by the claims appended therein.

## Claims

1. Vertebral osteosynthesis equipment, including bony anchoring members, such as pedicular screws (1) and/or lamar hooks, one or two linking rods (2), intended to be connected to these anchoring members and to be attached to the vertebrae by dint thereof, parts (3) for connecting this(these) linking rod (s) (2) to these anchoring members, and clamping means, such as nuts (4), for locking the linking rod (s) (2) in said connecting parts (3); at least one anchoring member is of the "polyaxial" type, i. e. comprises one proximal stud (5) articulated with respect to a body (6) intended for gripping a vertebra ; the proximal stud (5) of said polyaxial anchoring member exhibits an articulation head (11) in the form of a spherical cap; said body (6) of this anchoring member comprises a cavity (16) intended for accommodating this articulation head (11) and a wall (17) surrounding this cavity (16), this wall (17) being crimped around said articulation head (11), and said body (6) comprises a proximal gripping portion enabling to hold said body (6) when tightening said clamping means;
**characterized in that**:
- the spherical cap which said articulation head (11) forms has a height being less than the radius of the sphere containing the spherical wall of this articulation head (11);
- said wall (17) surrounding said cavity (16), after being crimped around said articulation head (11), is shaped in order to exhibit a proximal form in the form of a spherical cap, i.e. which height is less than the radius of the sphere containing the spherical wall of this wall (17), and
- said proximal gripping portion is formed of a collar (18) exhibiting several radial notches (19).

2. Vertebral osteosynthesis equipment according to claim 1 , **characterized in** the diameter of said articulation head (11) is of the order of double the diameter of said proximal stud (5).

3. Vertebral osteosynthesis equipment according to claim 1, **characterized in that** said collar (18) exhibits four notches (19) at 90 to one another.

4. Vertebral osteosynthesis equipment according to anyone of claims 1-3, **characterized in that** it comprises at least one connecting part (3) exhibiting a rounded section (20) intended for surrounding a linking rod (2) and two parallel drilled wings (21), these wings (21) being intended for engaging onto said proximal stud (5) and for being clamped, using said clamping means (4), against said crimped wall (17); the distal wing (21) of this connecting part (3) exhibits a distal cavity (28) in the form of a spherical cap, of greater diameter than that of said crimped wall (17), the connecting part (3) being intended for resting against this crimped wall (17) at this cavity (28).

5. Vertebral osteosynthesis equipment according to claim 4, **characterized in that** said clamping means is a nut (4) and the proximal branch (21) of this connecting part (3) comprises a proximal cavity (25) wherein a corresponding zone exhibited by this nut (4) is intended for engaging.

6. Vertebral osteosynthesis equipment according to claim 5, **characterized in that** said cavity (25) and said corresponding zone of the nut (4) are conical in shape.

7. Vertebral osteosynthesis equipment according to anyone of claims 1-6, **characterized in that** the stud (5) exhibits a zone (15) of reduced diameter, enabling to break its proximal portion after placing and clamping the nut (4).

8. Vertebral osteosynthesis equipment according to anyone of claims 1-7, **characterized in that** the proximal stud (5) and said connecting part (3) comprise means (14, 29) enabling to immobilise the proximal stud (5) in rotation when the connecting part (3) is engaged on this proximal stud (5).

9. Vertebral osteosynthesis equipment according to claim 8, **characterized in that** said means comprise at least one flat surface (14) provided on the proximal stud (5) and at least one flat surface provided on the connecting part (3), whereas these flats surfaces are immediately close to one another when the connecting part (3) is engaged on the proximal stud (5).

10. Method for manufacturing the polyaxial anchoring member according to anyone of claims 1 to 9, **characterized in that** it comprises the steps consisting in:
- providing, on the part intended for the proximal stud (5) of said polyaxial anchoring member, an articulation head (11) in the form of a spherical cap which height is less than the radius of the sphere containing the spherical wall of this articulation head (11);
- providing a cavity (16) in the proximal zone of the part intended for said body (6) of said anchoring member polyaxial, and, around this cavity (16), a wall (17) which may be crimped;
- engaging said articulation head (11) into said cavity (16),
- crimping said wall (17) around said articulation head (11) so that this wall (17) exhibits a proximal form in the form of a spherical cap which height is less than the radius of the sphere containing the spherical wall of this wall (17), and
- providing the proximal gripping portion as a collar (18) exhibiting several radial notches (19).

## Patentansprüche

1. Vertebrale Osteosynthesevorrichtung, aufweisend KnochenVerankerungsteile, wie Pedikelschrauben (1) und/oder Haken, ein oder zwei Verbindestäbe (2), die dazu vorgesehen sind, mit diesen Verankerungsteilen verbunden zu sein und an dem Wirbel mittels derselben befestigt zu sein, Teile (3) zum Verbinden dieses (dieser) Verbindestabes (Verbindestäbe) (2) mit diesen Verankerungsteilen, und Klemmmittel, wie Muttern (4), zum Arretieren des (der) Verbindestabes (Verbindestäbe) (2) an den Verbindeteilen (3); mindestens ein Verankerungsteil ist vom Typ "polyaxial", d.h. es weist einen bezüglich eines zum Greifen eines Wirbels vorgesehenen Körpers (6) artikulierten proximalen Bolzen (5) auf; der proximale Bolzen (5) des polyaxialen Verankerungsteils weist einen Artikulationskopf (11) in Form einer kugelförmigen Kappe auf; der Körper (6) dieses Verankerungsteils weist einen zum Unterbringen des Artikulationskopfs (11) vorgesehenen Hohlraum (16) und eine den Hohlraum (16) umgebende Wand (17) auf, diese Wand (17) ist um den Artikulationskopf (11) gecrimpt, und der Körper (6) weist einen proximalen Greifabschnitt auf, durch welchen der Körper (6) haltbar ist, wenn das Klemmmittel angezogen wird;
**dadurch gekennzeichnet, dass**:
- die kugelförmige Kappe, die der Artikulationskopf (11) formt, eine Höhe hat, die kleiner als der Radius der die kugelförmige Wand dieses Artikulationskopfs (11) beinhaltenden Kugel ist;
- die den Hohlraum (16) umgebende Wand (17), nachdem sie um den Artikulationskopf (11) gecrimpt ist, geformt ist, um eine proximale Form in Form einer kugelförmigen Kappe aufzuweisen, d.h. ihre Höhe ist kleiner als der Radius der die kugelförmige Wand dieser Wand (17) beinhaltenden Kugel, und
- der proximale Greifabschnitt von einem Kragen (18) mit mehreren radialen Einkerbungen (19) gebildet ist.

2. Vertebrale Osteosynthesevorrichtung gemäß Anspruch 1, **dadurch gekennzeichnet, dass** der Durchmesser des Artikulationskopfs (11) in der Größenordnung des zweifachen Durchmessers des proximalen Bolzens (5) ist.

3. Vertebrale Osteosynthesevorrichtung gemäß Anspruch 1, **dadurch gekennzeichnet, dass** der Kragen (18) vier Einkerbungen (19) in einem Winkel von 90° zueinander aufweist.

4. Vertebrale Osteosynthesevorrichtung gemäß einem der Ansprüche 1 bis 3, **dadurch gekennzeichnet, dass** sie mindestens ein Verbindeteil (3) mit einem gerundeten Abschnitt (20) aufweist, der vorgesehen ist einen Verbindestab (2) und zwei parallele gebohrte Flügel (21) zu umgeben, wobei diese Flügel (21) vorgesehen sind, um mit dem proximalen Bolzen (5) in Eingriff zu stehen und um mit dem Befestigungsmittel (4) gegen die gecrimpte Wand (17) geklemmt zu sein; der distale Flügel (21) des Verbindeteils (3) weist einen distalen Hohlraum (28) in Form einer kugelförmigen Kappe auf, deren Durchmesser größer als der der gecrimpten Wand (17) ist, wobei das Verbindeteil (3) vorgesehen ist, um an diesem Hohlraum (28) gegen die gecrimpte Wand (17) anzuliegen.

5. Vertebrale Osteosynthesevorrichtung gemäß Anspruch 4, **dadurch gekennzeichnet, dass** das Klemmmittel eine Mutter (4) ist und der proximale Arm (21) des Verbindeteils (3) einen proximalen Hohlraum (25) aufweist, wobei diese Mutter (4) einen entsprechenden Bereich aufweist, der zum in Eingriff stehen vorgesehen ist.

6. Vertebrale Osteosynthesevorrichtung gemäß Anspruch 5, **dadurch gekennzeichnet, dass** der Hohlraum (25) und der entsprechende Bereich der Mutter (4) in ihren Formen konisch sind.

7. Vertebrale Osteosynthesevorrichtung gemäß einem der Ansprüche 1 bis 6, **dadurch gekennzeichnet, dass** der Bolzen (5) einen Bereich (15) mit reduziertem Durchmesser aufweist, der es ermöglicht den proximalen Abschnitt des Bolzens (5) nach dem Platzieren und dem Klemmen der Mutter (4) zu brechen.

8. Vertebrale Osteosynthesevorrichtung gemäß einem der Ansprüche 1 bis 7, **dadurch gekennzeichnet, dass** der proximale Bolzen (5) und das Verbindeteil (3) ein Mittel (14, 29) aufweisen, welches es ermöglicht den proximalen Bolzen (5) in seiner Rotation zu immobilisieren, wenn das Verbindeteil (3) mit diesem proximalen Bolzen (5) in Eingriff steht.

9. Vertebrale Osteosynthesevorrichtung gemäß Anspruch 8, **dadurch gekennzeichnet, dass** das Mittel mindestens eine an dem proximalen Bolzen (5) bereitgestellte flache Oberfläche (14) und mindestens eine an dem Verbindeteil (3) bereitgestellte flache Oberfläche aufweist, wobei diese flachen Oberflächen sofort aneinander anliegen, wenn das Verbindeteil (3) mit dem proximalen Bolzen (5) in Eingriff steht.

10. Verfahren zum Herstellen des polyaxialen Verankerungsteils gemäß einem der Ansprüche 1 bis 9, **dadurch gekennzeichnet, dass** es folgende Schritte aufweist:
- Bereitstellen eines Artikulationskopfs (11) in Form einer kugelförmigen Kappe, deren Höhe kleiner als der Radius der die kugelförmige Wand des Artikulationskopfs (11) beinhaltenden Kugel ist, an dem Teil des polyaxialen Verankerungsteils, der für den proximalen Bolzen (5) vorgesehen ist;
- Bereitstellen eines Hohlraums (16) in dem proximalen Bereich des Teils, der für den Körper (6) des Verankerungsteils polyaxial vorgesehen ist, und einer Wand (17) um diesen Hohlraum (16), die gecrimpt sein kann;
- Eingreifen des Artikulationskopfs (11) in den Hohlraum (16),
- Crimpen der Wand (17) um den Artikulationskopf (11), so dass diese Wand (17) eine proximale Form in Form einer kugelförmigen Kappe aufweist, deren Höhe kleiner als der Radius der die kugelförmige Wand dieser Wand (17) beinhaltenden Kugel ist, und
- Bereitstellen des proximalen Greifabschnitts als einen Kragen (18), der eine Mehrzahl an radialen Einkerbungen (19) aufweist.

## Revendications

1. Matériel d'ostéosynthèse vertébrale, comprenant des organes d'ancrage osseux, tels que des vis pédiculaires (1) et/ou crochets lamaires, une ou deux tiges de liaison (2), destinées à être reliées à ces organes d'ancrage et à être fixées aux vertèbres au moyen de ceux-ci, des pièces de connexion (3) de cette ou ces tiges de liaison (2) à ces organes d'ancrage, et des moyens de serrage, tels que des écrous (4), pour bloquer la ou les tiges de liaison (2) dans lesdites pièces de connexion (3) ; au moins un organe d'ancrage est du type "polyaxial", c'est-à-dire comprend un pion proximal (5) articulé par rapport à un corps (6) destiné à venir en prise avec une vertèbre ; le pion proximal (5) dudit organe d'ancrage polyaxial présente une tête d'articulation (11) sous la forme d'une calotte sphérique ; le corps (6) de cet organe d'ancrage comprend une cavité (16) destinée à recevoir cette tête d'articulation (11) et une paroi (17) entourant cette cavité (16), cette paroi (17) étant sertie autour de ladite tête d'articulation (11), et ledit corps (6) comprenant une partie proximale de préhension permettant de maintenir ledit corps (6) lors du serrage desdits moyens de serrage ;
**caractérisé en ce que** :
- la calotte sphérique que forme la tête d'articulation (11) a une hauteur inférieure au rayon de la sphère contenant la paroi sphérique de cette tête d'articulation (11) ;
- ladite paroi (17) entourant ladite cavité (16), après avoir été sertie autour de ladite tête d'articulation (11), est conformée de manière à présenter une forme proximale en calotte sphérique, c'est-à-dire dont la hauteur est inférieure au rayon de la sphère contenant la paroi sphérique de cette paroi (17), et
- ladite partie proximale de préhension est formée par une collerette (18) présentant plusieurs encoches radiales (19).

2. Matériel d'ostéosynthèse vertébrale selon la revendication 1, caractérisé en ce le diamètre de ladite tête d'articulation (11) est de l'ordre du double du diamètre dudit pion proximal (5).

3. Matériel d'ostéosynthèse vertébrale selon la revendication 1, **caractérisé en ce que** ladite collerette (18) présente quatre encoches (19) à 90° les unes des autres.

4. Matériel d'ostéosynthèse vertébrale selon l'une quelconque des revendications 1 à 3, **caractérisé en ce qu'**il comprend au moins une pièce de connexion (3) présentant une partie arrondie (20) destinée à entourer une tige de liaison (2) et deux ailes parallèles (21) percées de trous, ces ailes (21) étant destinées à être engagées sur ledit pion proximal (5) et à être serrées, à l'aide dudit moyen de serrage (4), contre ladite paroi sertie (17) ; l'aile distale (21) de cette pièce de connexion (3) présente une cavité distale (28) en forme de calotte sphérique, de diamètre supérieur à celui de ladite paroi sertie (17), la pièce de connexion (3) étant destinée à venir reposer contre la paroi sertie (17) au niveau de cette cavité (28).

5. Matériel d'ostéosynthèse vertébrale selon la revendication 4, **caractérisé en ce que** ledit moyen de serrage est un écrou (4) et **en ce que** la branche (21) proximale de cette pièce de connexion (3) comprend une cavité proximale (25) dans laquelle est destinée à être engagée une zone correspondante que présente cet écrou (4).

6. Matériel selon la revendication 5, **caractérisé en ce que** ladite cavité (25), et ladite zone correspondante de l'écrou (4) ont une forme conique.

7. Matériel selon l'une des revendications 1 à 6, **caractérisé en ce que** le pion (5) présente une zone (15) de diamètre réduit, permettant de casser la portion proximale de ce pion (5) après mise en place et serrage du moyen de serrage (4).

8. Matériel selon l'une des revendications 1 à 6, **caractérisé en ce que** le pion (5) et la pièce de connexion (3) comprennent des moyens (14, 29) permettant d'immobiliser le pion (5) en rotation lorsque la pièce de connexion (3) est engagée sur ce pion (5).

9. Matériel selon la revendication 8, **caractérisé en ce que** lesdits moyens comprennent au moins un méplat (14) aménagé sur le pion (5) et au moins un méplat aménagé sur la pièce de connexion (3), ces méplats venant à proximité immédiate l'un de l'autre lorsque la pièce de connexion (3) est engagée sur le pion (5).

10. Procédé de fabrication de l'organe d'ancrage polyaxial selon l'une des revendications 1 à 9, **caractérisé en ce qu'**il comprend les étapes consistant à :
- aménager, sur la pièce destinée à constituer le pion proximal (5) dudit organe d'ancrage polyaxial, une tête d'articulation (11) en forme de calotte sphérique dont la hauteur est inférieure au rayon de la sphère contenant la paroi sphérique de cette tête d'articulation (11) ;
- aménager une cavité (16) dans la zone proximale de la pièce destinée à constituer ledit corps (6) dudit organe d'ancrage polyaxial, et, autour de cette cavité (16), une paroi (17) susceptible d'être sertie ;
- engager ladite tête d'articulation (11) dans ladite cavité (16) ;
- sertir ladite paroi (17) autour de ladite tête d'articulation (11) de telle sorte que cette paroi (17) présente une forme proximale au moins partiellement hémisphérique dont la hauteur est inférieure au rayon de la sphère contenant la paroi sphérique de cette paroi (17), et
- aménager la partie proximale de préhension sous la forme d'une collerette (18) présentant plusieurs encoches radiales (19).
